# EUROPEAN PATENT SPECIFICATION

(11) **EP 1 194 037 B1**
(45) Date of publication and mention of the grant of the patent: **18.08.2004**
(21) Application number: 00948095.5
(22) Date of filing: 12.07.2000
(51) Int. Cl.: A01N 25/30, C11D 1/75, C11D 1/72, C11D 1/62, C07C 217/52, C07C 217/44, C07C 217/28, C07C 217/08, C07C 291/04, C07C 215/44, C07C 215/40, C07C 215/18, C07C 215/12, C07C 217/42, A01N 57/20, A01N 43/40, A01N 43/653

(54) **AGROCHEMICAL COMPOSITIONS AND SURFACTANT COMPOUNDS**
AGROCHEMISCHE ZUSAMMENSETZUNGEN UND TENSIDVERBINDUNGEN
COMPOSITIONS AGROCHIMIQUES ET COMPOSES TENSIOACTIFS

(30) Priority: 16.07.1999 GB 9916581; 21.08.1999 GB 9919790
(43) Date of publication of application: 10.04.2002
(73) Proprietor: Imperial Chemical Industries PLC, London W1U 3AN (GB)
(72) Inventor: BEVINAKATTI, Hanamanthsa, Shankarsa, Yarm, Cleveland TS15 9EW (GB); REEKMANS, Steven, Irene, Jozef, 1060 Brussels (BE); SCOVELL, Edward, George, Middlesbrough, Cleveland TS9 6DS (GB); BLEASE, Trevor, Graham, Stockton on Tees, Cleveland TS19 7SP (GB); DAVIES, Simon, John, Guisborough, Cleveland TS14 8HT (GB)
(74) Representative: Roberts, Jonathan Winstanley
(86) International application number: PCT/GB2000/002670
(87) International publication number: WO 2001/005224

(56) References cited:
- EP-A- 0 206 998
- WO-A-93/05763
- WO-A-96/32839
- DE-A- 4 238 214
- DE-A- 4 238 216
- CHEMICAL ABSTRACTS, vol. 96, no. 8, 22 February 1982 (1982-02-22) Columbus, Ohio, US; abstract no. 57579, K. HONDA ET AL.: "Properties, application and NMR spectroscopic study of some new nonionic surface active agents useful in formulation of W/O-emulsions. " XP002149882 & K. HONDA ET AL.: "-as above- " J. SOC. COSMET. CHEM., vol. 32, no. 5, 1981, pages 255-273,

## Description

This invention relates to agrochemical compositions including surfactant compounds which include a polyhydroxy hydrocarbyl, particularly a saccharide, amine residue, a hydrophobic residue and a linking group, particularly including a glycidyl group, to the use of such compounds as surfactants in agrochemicals and to certain of these compounds as such.

Surfactants are widely used in agrochemical compositions and formulations for a variety of reasons including as adjuvants, wetting agents, emulsifiers or solubilisers (or to serve more than one such function). Adjuvants act to increase the effect of agrochemicals (by a variety of possible mechanisms); wetting agents improve the wetting of agrochemical sprays on the target substrate, usually plant leaves; emulsifiers are used to emulsify liquid agrochemicals in aqueous media, to emulsify oils used as solvents or diluents for agrochemicals and/or to emulsify oils used as formulation additives (to provide improved properties); and solubilisers are used to improve the solubility or compatibility of otherwise insoluble or incompatible formulation components. The benefit of including surfactants in agrochemical formulations is widely recognised and for many agrochemicals is a very widespread practice.

Surfactants including polyhydroxy hydrocarbyl, particularly saccharide, substituents, particularly as amides have been suggested e.g. for cleaning applications. Other surfactant compounds including polyhydroxy hydrocarbyl and amino groups are disclosed in JP 54163829 A to fatty alcohol glycidyl amine glucoside derivatives in making cosmetic emulsions; DE 4238214 A and DE 4238215 A to fatty glycidyl amine glucoside derivatives in making polyurethane materials; DE 4238216 A and

DE 4238217 A to quaternary derivatives of such materials as textile surfactants and DE 4307475 A to betaine derivatives.

WO 96/32839 A2 discloses glyphosate formulations comprising polyetheramine surfactants having low eye irritancy properties.

Honda et al. disclose in J. Soc. Cosmet. Chem. Vol. 32, 255- 273 (1981), low toxic N-hydroxypropyl-alkanolamines as advantageous surfactants for cosmetic emulsions.

This invention is based on the finding that certain surfactants including polyhydroxy hydrocarbyl, particularly saccharide, amine groups can be useful in agrochemical applications, compositions and formulations, in particular providing adjuvancy, wetting, emulsification, dispersancy, thickening and/or solublisation. The compounds of and used in this invention can:
1 provide enhanced activity for agrochemicals, especially water soluble herbicides, notably in terms of enhancing the speed of effectiveness; and
2 have significantly lower aquatic toxicity than conventional surfactants used in agrochemical formulations, especially adjuvant surfactants.

The present invention accordingly provides an agrochemical composition which includes an agrochemically active compound and a compound of the formula (I):

R¹ - (R²)X¹ - [Link]- R³ (I)

where
- R¹: is polyhydroxy hydrocarbyl;
- R²: is H or hydrocarbyl, particularly alkyl, hydroxyalkyl or alkoxyalkyl, or is a group as defined for R¹;
- X¹: is N; N⁺->O⁻; N⁺R⁴ - where: R⁴ - is C₁ to C₆ hydrocarbyl carrying an anionic substituent, particularly - CH₂ - COO⁻; or N⁺R⁵ An- where: R⁵ is a C₁ to C₂₀ hydrocarbyl, particularly alkyl, hydroxyalkyl, alkoxyalkyl or aralkyl; and An⁻ is a charge balancing anion e.g. alkali metal or ammonium;
- Link: is a linking group of the formula: - CH₂ - CHOH - X² -
where X² is a direct bond; - CH₂ - O -; - CH₂ - N(R⁶) -; - CH₂ - (OA)ₚ- O -; or
- CH₂- (OA)ₚ- N(R⁷)-;
where
OA is an oxyalkylene residue;
p is from 1 to 100;
R⁶ is H; C₁ to C₈ hydrocarbyl, especially alkyl or alkenyl; or a group R¹ - (R²)X¹ - CH₂- CHOH - CH₂ - where R¹, R² and X¹ are as defined above; and
R⁷ is H; C₁ to C₈ hydrocarbyl, especially alkyl or alkenyl; or a group R¹ - (R²) X¹ - CH₂- CHOH - CH₂- (OA)p- where R¹, R², X¹, OA and p are as defined above; and
- R³: is hydrocarbyl, usually C₆ to C₃₀, particularly C₆ to C₃₀, more particularly C₁₀ to C₃₀, especially alkyl, alkenyl, alkaryl, aryl or aralkyl.

The invention also includes compounds of the formula (IIa) [within the general formula (I)]:

R¹ - (R2) X¹ - [Link¹] - R³ (IIa)

where R¹, R², and R³ are as defined above for formula (I); and
Link¹ is a linking group of one of the formulae:

- CH₂ - CHOH - CH₂ - (OA)p- O -;

- CH₂ - CHOH - CH₂ - N(R⁶) -;

or

- CH₂ - CHOH - CH₂ - (OA)p- N(R⁷) -;

where OA, p, R⁶ and R⁷ are as defined above for formula (I).

The invention further specifically includes compounds of the formula (IIb) [within the general formula (I)):

R¹ - (R²)X^{1a}- [Link²] - R³ (IIb)

where R¹, R², and R³ are as defined above for formula (I);
X^{1a} is N+->O⁻, N+R^{4 -} or R⁵ An⁻ where: R⁴⁻, R⁵ and An- are as defined above for formula (I); and
Link² is a linking group of one of the formulae:

- CH₂ - CHOH - CH₂ - O -;

- CH₂ - CHOH - CH₂ - (OA)p- O -;

- CH₂ - CHOH - CH₂ - N(R⁶) -;

or

- CH₂ - CHOH - CH₂ - (OA)p- N(R⁷) -;

where OA, p, R⁶ and R⁷ are as defined above for formula (I).

The invention particularly includes agrochemical compositions which include an agrochemically active compound and, particularly as an adjuvant, at least one compound of at least one of the formulae (IIa) or (IIb). The invention further includes the use of compounds of any of the formulae (I), (IIa) or (IIb) as agrochemical surfactants, particularly as adjuvants.

The group R¹ is a polyhydroxy hydrocarbyl, particularly polyhydroxy alkyl, group, and desirably has a linear C₄ to C₇ chain and at least three hydroxyl groups directly bonded to chain carbon atoms. The group may include substituents, in particular, alkoxy groups e.g. by etherification of further hydroxyl groups or further polyhydroxy hydrocarbyl, e.g. polyhydroxy alkyl, group(s), but the group desirably includes at least three free hydroxyl groups including such hydroxyl groups on substituents of the basic chain. Particularly R¹ is an open chain tetratol, pentitol, hexitol or heptitol group or an anhydro e.g. cycloether anhydro, derivative of such a group. Especially desirably, R¹ is the residue of, or a residue derived from, a sugar, particularly a monosaccharide such as glucose, fructose or sorbitol, a disaccharide such as maltose or palitose or a higher oligosaccharide. It is particularly convenient that R¹ is the residue of a reducing sugar, because the amines can be made by straightforward reductive alkylation reactions on ammonia or an amine H₂NR².

In the compounds of the formula (I) of and used in this invention the group R¹ is present as or as part of the hydrophile. Thus it will usually be desirable that the hydrophilicity of this group is not unduly reduced. The open chain form of such groups is typically the most hydrophilic form and will thus usually be the form desired. Groups including internal cyclic ether functionality can however be used, if desired, and may be obtained inadvertently if the synthetic route exposes the group to relatively high temperatures or other conditions which promote such cyclization.

Where R¹ is the residue of, or a residue derived from, a monosaccharide, the saccharide derived group or residue will usually be present as an open chain material. Where R¹ is the residue of, or a residue derived from, an oligosaccharide it can be considered as an open chain monosaccharide derived group or residue with a saccharide or oligosaccharide substituent which may be cyclic or a chain of cyclic residues. Particularly useful R¹ groups are derived from glycoses and are of the formula: -CH₂ - (CHOH)₄ - CH₂OH, e.g. corresponding to residues from glucose, mannose or galactose. In this case the group -NR¹ R² is of the formula: - NR²-CH₂-(CHOH)₄-CH₂OH and the group is conveniently called a glycamine group. Most commonly the group R¹ will be derived from glucose and the corresponding amines may be are called glucamines (as they will usually be made from glucose) or sorbitylamines (as they are no longer unsaturated). Strictly, such compounds are derivatives of 1-deoxyglycitols (and 1-deoxyglucitols) and can be referred to as 1-deoxyglycitylamines (and 1-deoxyglucitylamines) or as corresponding aminoglycitols (and aminoglycitols).

The group X¹ is a nitrogen atom which either has no further substituent (other than R¹, R² and Link) or includes a substituent which makes the group a quaternary group, so that when X¹ is a substituted nitrogen atom it can be an amine oxide group N-->O; a group N⁺R⁴ -; or N⁺R⁵ An⁻.

When X¹ is a nitrogen atom, the substituent R² on the nitrogen atom of X¹, can be a hydrocarbyl group (see further beiow) or it can be as defined for R¹ in which case the amine function provides two hydrophilic polyhydroxy hydrocarbyl groups. In this case, the two groups of the formula R¹ will often be (but need not be) the same, as it usually easier to make the symmetrical polyhydroxy hydrocarbyl substituted amine intermediate.

Where the group R² is a hydrocarbyl group, it is desirably an alkyl or alkenyl group, and typically it has from 1 to 30, more usually from 1 to 22, carbon atoms. R² can be a blocking group (mainly used to keep the synthesis straightforward), as when R² is a lower e.g. C₁ to C₆, alkyl group, particularly a methyl or ethyl group. R² can be a longer chain e.g. C₆ to C₃₀, particularly a C₈ to C₂₂ alkyl, group and such a longer chain group will tend to act as a secondary hydrophobe. R² can also be a substituted alkyl group e.g. a hydroxy or alkoxy substituted alkyl group, particularly a C₂ to C₆ alkyl group which is hydroxy substituted e.g. a hydroxyethyl, particularly 2-hydroxyethyl, or hydroxypropyl, particularly 3-hydroxypropyl, group, or a C₁ to C₆ alkyl group substituted with an alkoxy, particularly a C₁ to C₆ alkoxy and especially a methoxy, ethoxy or propoxy, group, so that the alkoxyalkyl group is particularly a 2-methoxyethyl, 2-ethoxyethyl, 3-methoxypropyl, or 3-ethoxypropyl group. The additional hydroxyl group or oxygen atom may provide a modest increase in water solubility. R² can also be an aralkyl group, particularly a C₇ to C₁₂ aralkyl group, such as a benzyl group.

When X¹ is a group N⁺R⁴ -, the group R⁴ is a C₁ to C₆ hydrocarbyl group carrying an anionic substituent (nominally carrying a balancing negative charge). Thus, typically R⁴ - is a carboxyalkyl group, particularly a -CH₂-COO⁻ group forming a betaine structure, although other possibilities include, alkyl sulphate, alkyl sulphonate, alkyl phosphate and alkyl phosphonate groups. The precise charge status and the presence of other ions associated with such groups will depend mainly on the pH. At near neutrality, the compound is likely to exist mainly as the zwitterion, whereas remote from neutrality, the quaternary nitrogen and the anionic group in R⁴ may become associated with charge balancing ions. The charge balancing ions will usually be alkali metal or onium (ammonium or amine onium) ion for the anionic, usually carboxyl, group and halide, sulphate, phosphate or carboxylic acids for the amine function.

When X¹ is a group N⁺R⁵ An-, the group R⁵ is a C₁ to C₂₂ hydrocarbyl, particularly an alkyl group and more usually a C₁ to C₆ or a C₁₀ to C₁₈ alkyl group, a C₂ to C₆ hydroxy alkyl group, a (C₁ to C₆)alkoxy (C₁ to C₆)alkyl group or a C₇ to C₁₂ aralkyl, particularly a benzyl, group. Where R⁵ is an alkyl group, it will most commonly be a C₁ to C₆ alkyl, particularly methyl, group, although it may be a longer chain e.g. C₆ to C₃₀, particularly a C₈ to C₂₂ alkyl, group and such a longer chain group will tend to act as a secondary hydrophobe. The anion group An- is a charge balancing anion and can be any suitable counterion, for example mineral acid anions such as a halide, particularly chloride or bromide, sulphate or phosphate ion or a fatty carboxylate species.

The group Link is a group - CH₂ - CHOH - X² - group which functions to connect the hydrophilic substituted amino group with the hydrophobic group R³. As such its precursor(s) provide suitable reactivity to enable the '"linking" reactions but desirably do not include functionality that would interfere with the desired properties of the end products. The Link groups include a hydroxyl group, typically derived from epoxy or glycidyl functionality in synthetic precursors, which may provide a modest increase in the hydrophilicity of the end product. The group Link can be connected to the group R³ by a direct bond, as when the corresponding precursor is a 1-epoxy hydrocarbyl, particularly alkyl, compound; an oxygen atom, an amino function or a (poly)alkylenoxy chain which itself may be linked to the group R³ through an oxygen atom or an amino function. Where the group Link includes an amino function, the amino group may be substituted with a residue that includes a further glycidyl group (linked as appropriate *via* a (poly) alkyleneoxy chain) and a hydrophilic (polyhydroxy hydrocarbyl)amino residue. In such compounds, the further glycidyl linked group is desirably the same as the first glycidyl linked group in the molecule. The group Link is desirably a group as defined for Link¹ or Link² in formulae (IIa) and (IIb) above i.e. it is desirably a group of one of the formulae: - CH₂ - CHOH - CH₂ - O -;- CH₂ - CHOH - CH₂ - (OA)ₚ- O - ; - CH₂ - CHOH - CH₂ - N(R⁶)-; or - CH₂ - CHOH - CH₂ - (OA)ₚ- N(R⁷) -; where OA, p, R⁶ and R⁷ are as defined above.

When the linking group includes an oxyalkylene group or chain - (OA)ₚ-, the oxyalkylene group(s) can be oxyethylene (- C₂H₄- O -), oxyproylene (- C₃H₆- O -) or oxybutylene (- C₄H₈- O -), but desirably the oxyalkylene groups are all oxyethylene groups or are mixtures of oxyethylene and oxypropylene groups, desirably having a molar ratio of oxyethylene to oxypropylene groups of from 1:5 to 10:1. When the oxyalkylene groups are mixed oxyethylene and oxypropylene groups, the polyoxyalkylene chain can be a random or block copolymeric chain. Within the range 1 to 100, p is desirably 1 to 50, particularly 1 to 30. The number of units in the (poly)oxyalkylene chain, 'p', is an average value and may be non-integral.

The groups R⁶ and R⁷ can be C₁ to C₈ hydrocarbyl, particularly alkyl or alkenyl, groups. More usually they will be groups corresponding to the hydrophile linked to the Link group and will thus be R¹ - (R²)X¹ - CH₂- CHOH - CH₂- for R⁶ and R¹ - (R²) X¹ - CH₂- CHOH - CH₂- (OA)ₚ- for R⁷.

The group R³ is or contains a hydrophobic hydrocarbyl group, particularly an alkyl or alkenyl group. R³ may be a straight chain group or may be branched or a mixture of straight chain and branched moieties. Where the hydrophobic group is connected to the link group by a direct bond or an ether group (including a polyoxyalkylene ether group) the hydrocarbyl radical is desirably an alkyl or alkenyl group. Generally it is a C₆ to C₃₀, usually C₈ to C₃₀, more usually a C₁₀ to C₃₀, particularly a C₁₂ to C₂₀, especially a C₁₂ to C₁₈, group. R³ may also be an alkyl phenyl group e.g. a C₈ to C₁₈ alkyl phenyl group and particularly a 3-linear alkyl phenyl group. Such groups can be derived from cardenols (3-alkyl phenols) which are readily biodegradeable compounds.

In particular the invention is directed to the compounds of the formulae (IIIa) to (IIIs) [including (IIIg') to (IIIs')] and/or their use in agrochemical compositions and formulations, particularly as adjuvants:

R¹ - (R²)N - CH₂ - CHOH - R³ (IIIa)

(R¹)₂N - CH₂ - CHOH - R³ (IIIb)

compounds of the formulae (IIIa) and (IIIb) are compounds of the formula (I) where X¹ is a nitrogen atom and Link is a group: - CH₂ - CHOH - ;

R¹ - (R²)N - CH₂ - CHOH - CH₂ - O - R³ (IIIc)

(R¹)₂N - CH₂ - CHOH - CH₂ - O - R³ (IIId)

compounds of the formulae (IIIc) and (IIId) are compounds of the formula (I) where X¹ is a nitrogen atom and Link is a group: - CH₂ - CHOH - CH₂ - O -;

R¹ - (R²)N - GH₂ - CHOH - GH₂ - (OA)p- O - R³ (IIIe)

(R¹)₂N - CH₂ - CHOH - CH₂ - (OA)p- O - R³ (IIIf)

compounds of the formulae (IIIe) and (IIIf) are compounds of the formula (I) where X¹ is a nitrogen atom and Link is a group: - CH₂ - CHOH - CH₂ - (OA)ₚ- O - ;

R¹ - (R²)N - CH₂ - CHOH - CH₂ - N(R⁶) - R³ (IIIg)

(R¹)₂N - CH₂ - CHOH - CH₂ - N(R⁶) - R³ (IIIh)

and particularly (IIIg') and (IIIh'):

[R¹ - (R²)N - CH₂ - CHOH - CH₂]₂- N - R³ (IIIg')

[(R¹)₂N - CH₂ - CHOH - CH₂]₂- N - R³ (IIIh')

compounds of the formulae (IIIg) and (IIIh) are compounds of the formula (I) where X¹ is a nitrogen atom and Link is a group: - CH₂ - CHOH - CH₂ - N(R⁶) -;

R¹ - (R²)N - CH₂ - CHOH - CH₂ - (OA)ₚ- N(R⁷) - R³ (IIIj)

(R¹)₂N - CH₂ - CHOH - CH₂ - (OA)ₚ- N(R⁷) - R³ (IIIk)

and particularly (IIIj') and (IIIk'):

[R¹ - (R²)N - CH₂ - CHOH - CH₂ - (OA)ₚ]₂- N - R³ (IIIj')

[(R¹)₂N - CH₂ - CHOH - CH₂ - (OA)ₚ]₂- N - R³ (IIIk')

compounds of the formulae (IIIj) and (IIIk) are compounds of the formula (I) where X¹ is a nitrogen atom and Link is a group: - CH₂ - CHOH - CH₂ - (OA)ₚ- N(R⁷) -;

R¹ - (R²)(N->O) - Link - R³ (IIIm)

(R¹)₂(N->O) - Link - R³ (IIIn)

and particularly (IIIm') and (IIIn')

R¹ - (R²)(N->O) - Link² - R³ (IIIm')

(R¹)₂(N->O) - Link²- R³ (IIIm')

compounds of the formulae (IItm) and (IIIn) are compounds of the formula (I) where X¹ is an amine oxide group;

R¹ - (R²)N⁺R^{4 -}- Link - R³ (IIIp);

(R¹)₂N⁺R^{4 -}- Link - R³ (IIIq);

and particularly (IIIp') and (IIIq')

R¹ - (R²)N⁺R⁴ -- Link²⁻ R³ (IIIp')

(R¹)₂N⁺R^{4 -}- Link²⁻ R³ (IIIq')

compounds of the formula (IIIp) and (IIIq) are compounds of the formula (I) where X¹ is a quaternary nitrogen atom and a substituent group including anionic functionality;

R¹ - (R²)N⁺R⁵ An⁻- Link - R³ (IIIr)

(R¹)₂N⁺R⁵ An⁻- Link - R³ (IIIs)

and particularly (IIIr') and (IIIs')

R¹- (R²)N⁺R⁵ An⁻- Link²- R³ (IIIr')

(R¹ )₂N⁺R⁵ An⁻- Link²- R³ (IIIs')

compounds of the formula (IIIr) and (IIIs) are compounds of the formula (I) where X¹ is a quaternary nitrogen atom with a charge balancing anion.

In the formulae (IIIa) to (IIIs) [including (IIIg') to (IIIs')] each R¹, R², R³, R⁴, R⁵, R⁶, R⁷, An, Link, OA, and p is independently as defined for formula (I).

The invention includes agrochemical compositions which includes an agrochemically active compound and, particularly as an adjuvant, at least one compound of at least one of the formulae (IIIa) to (IIIs) [including (IIIg') to (IIIs')]. The invention further includes the use of compounds of any of the formulae (IIIa) to (IIIs) [including (IIIg') to (IIIs')] as agrochemical surfactants, particularly as adjuvants.

The compounds of and used in the invention can be made by routes involving generally conventional synthetic steps. In particular:
Compounds of the formulae (IIIa) and (IIIb) can be made by reacting an amine (IV): R¹R²NH (IV) with an epoxide (V): 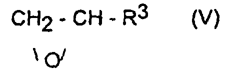 under nucleophilic epoxide ring opening conditions.
Compounds of the formulae (IIIc) and (IIId) can be made by reacting an amine (IV) (as above) with a glycidyl ether (VIa): 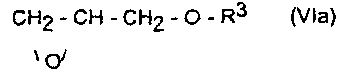 under nucleophilic epoxide ring opening conditions.
Compounds of the formulae (IIIe) and (IIIf) can be made by reacting an amine (IV) (as above) with a glycidyl ether (VIb): 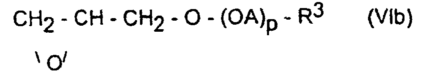 under nucleophilic epoxide ring opening conditions.
Compounds of the formulae (IIIg) and (IIIh) can be made by reacting an amine (IV) (as above) with a glycidyl amine (VIc): 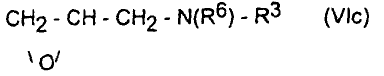
Compounds of the formulae (IIIg') and (IIIh') can be made by reacting two moles of an amine (IV) (as above) with one mole of a bis-glycidylamine (Vlc'): 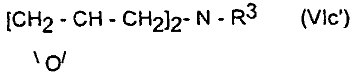
Compounds of the formulae (IIIj) and (IIIk) can be made by reacting an amine (IV) (as above) with a glycidyl ether amine (VId): 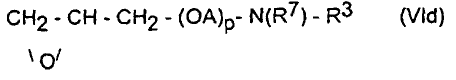 under nucleophilic epoxide ring opening conditions.
Compounds of the formulae (IIIj') and (IIIk') can be made by reacting two moles of an amine (IV) (as above) with one mole of a bis-glycidylamine (VId'): 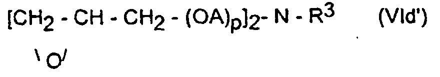 under nucleophilic epoxide ring opening conditions.
Compounds of the formulae (IIIm) and (IIIn) can be made by oxidising e.g. with hydrogen peroxide, an amine of the formula: R¹ - (R²)N - Link - R³.
Compounds of the formulae (IIIp) and (IIIq) can be made by reaction of an amine of the formula: R¹ - (R²)N - Link - R³ with a reactive precursor of the group R^{4 -}, typically a halogen derivative, under nucleophilic substitution conditions.
Compounds of the (IIIr) and (IIIs) can be made by reaction of an amine of the formula: R¹ - (R²)N - Link - R³ with a quaternizing, usually an alkylating, agent.

In the above outline reaction sequences the groups R¹, R², R³, R⁴, R⁵, R⁶, R⁷, Link, OA and p are as defined above.

Typically, reactions of epoxides and amines in the syntheses outlined above are carried out by heating the reagents in solution or dispersion in an inert solvent or diluent (glycols such as monopropylene glycol are suitably inert for this purpose). Compounds where X¹ is a substituted nitrogen atom can be made from the corresponding compounds where X¹ is an unsubstituted nitrogen atom by reaction with a suitable reactive intermediate, particularly a halogen substituted compound including the residue for substitution on the nitrogen atom.

Precursors used above can be made by the following general routes:
Amines of the formula (IV) (R¹R²NH) can be made by reductive alkylation of an amine R²NH with a reactive precursor of the residue R¹, e.g. a reducing sugar of which R¹H is a (possibly notional) 1-deoxy derivative.
Epoxides of the formula (V) can be made by selective oxidation of olefins of the formula: CH₂ = CH - R³.

Glycidyl ethers of the formulae (VIa) and (VIb) can be made by reacting alcohols of the formulae R³OH and R³ - (OA)ₚ- OH respectively with *epi*-chlorohydrin under nucleophilic substitution conditions (of course avoiding conditions that promote epoxide ring opening).

Glycidyl amines of the formulae (VIc) and (VIc') can be made by reacting.amines of the formulae: HN(R⁶) R³ and H₂N - R³ with *epi*-chlorohydrin under nucleophilic substitution conditions.

Glycidyl ether amines of the formulae (VId) and (VId') can be made by alkoxylating amines of the formulae: HN(R⁷) - R³ and H₂N - R³ and subsequently reacting the product (poly)alkyleneoxyamines with *epi*-chlorohydrin under nucleophilic substitution conditions.

The compounds of the formula (I) above can be used in agrochemical formulations particularly as adjuvants, emulsifiers, wetting agents, dispersants, thickeners or solubilisers and the invention accordingly includes agrochemical formulations incorporating compounds of the formula (I), particularly formulae (IIa) and (IIb) or formulae (IIIa) to (IIIs), as adjuvants, emulsifiers, wetting agents, dispersants, thickeners or solubilisers.

Surfactants of the formula (I) and particularly of the formulae (IIa), (IIb) or (IIIa) to (Ills) can be used (particularly as adjuvants) with a wide range of agrochemical active materials and specifically, the active component of the formulation may be one or more plant growth regulators, herbicides, and/or pesticides, for example insecticides, fungicides, acaricides, nematocides, miticides, rodenticides, bactericides, molluscicides and bird repellants. Specific examples of actives include:
Herbicides: including
   water soluble, particularly non-selective, herbicides, more particularly phosphonomethyl glycines, especially as salts such as Glyphosate and Sulfosate {respectively the *iso*-propylamino and trimethylsulphonium salts of N-phosphonomethyl glycine}; and phosphinyl amino acids such as Glufosinate {2-amino-4-(hydroxymethylphosphinyl) butanoic acid} particularly as the ammonium salt and bipyridinium compounds such as Paraquat {1,1'-dimethyl-4,4'-bipyridinium};
   triazines such as Atrazine {6-chloro-*N*-ethyl-N'-(1-methylethyl)-1,3,5-triazine-2,4-diamine, and Prometryn {*N,N'*-bis(1-methylethyl)-6-(methylthio)-1,3,5-triazine)-2,4-diamine};
   substituted ureas such as Diuron {*N*'-(3,4-dichlorophenyl)-*N,N*-dimethylurea};
   sulphonyl ureas such as metsulfuron-methyl {2-[[[[(4-methoxy-6-methyl-1,3,5-triazin-2-yl) amino}carbonyl]amino)sulfonyl]benzoate}, triasulfuron {2-(2-chloroethoxy)-N-[[(4-methoxy-6-methyl-1,3,5-triazin-2-yl)amino]carbonyl]benzenesulfonamide}, tribenuron-methyl {methyl 2-[[[[(4-methoxy-6-methyl-1,3,5-triazin-2-yl)-methylamino]carbonyl]aminolsulfonyl] benzoate} and chlorsulfuron {2-chloro-*N*-[[(4-methoxy-6-methyl-1,3,5-triazin-2-yl)amino]carbonyl] benzenesulfonamide};
   pyridine carboxylic acids such as clopyralid {3,6-dichloropyridine-2-carboxylic acid};
   aryloxy alkanoic acids such as 2,4-D {2,4-dichlorophenoxyacetic acid};
   2-(4-aryloxyphenoxy)propionic acids such as clodinafoppropargyl {prop-2-ynil (R)-2-[4-(5-chloro-3-fluoropyridinr-2-yloxy) phenoxy]-propionate}; and
   bis-carbamates such as Phenmedipham {3-[(methoxycarbonyl)amino]phenyl (3-methyl phenyl)carbamate}.
Fungicides: including
   thiocarbamates, particularly alkylenebis(dithiocarbamate)s, such as Maneb {[1,2-ethanediylbis-[carbamodithiato](2-)]manganese} and Mancozeb {[[1,2-ethanediylbis[carbamodithiato]](2-)]manganese mixture with ([1,2-ethanediylbis(carbamodithiato]] (2-)]zinc};
   strobilurins such as azoxystrobin {methyl (E)-2-[[6-(2-cyanophenoxy)-4-pyrimidinyl]oxy]-a-(methoxymethylene)benzeneacetate) and kresoxim-methyl {(E)-a-(methoxyimino)-2-[(2-methylphenoxy)methyl]benzeneacetic acid methyl ester);
   dicarboximides such as Iprodione {3-(3,5-dichlorophenyl)-N-isopropyl-2,4-dioxo imidazolidine-1-carboxamide} ;
   halogenated phthalonitriles such as 2,4,5,6-tetrachloro-1,3-dicyanobenzene;
   benzimidazoles such as Carbendazym {methyl benzimidazol-2-yl carbamate};
   azoles such as Propiconazole {1-[2-(2,4-dichlorophenyl)-4-propyl-1,3-dioxotan-2-yl-methyl-1 H-1,2,4-triazole}, and Tebuconazole {(RS)-1-p-chlorophenyl-4,4-dimethyl-3-(1 H-1,2,4-triazote-1-ylmethyl)-pentan-3-ol}; and
   inorganic fungicides such as Copper hydroxide {Cu(OH)2);
   benzoyl ureas such as Diflubenzuron {N-[[(4-chlorophenyl)amino]carbonyl]-2,6-difluorobenzamide)} and pyrethroid insecticides; and

Acaricides including: tetrazines such as Clofentezine {3,6-bis(2-chlorophenyl)-1,2,4,5-tetrazine}.

Among water soluble active materials particularly suitable actives include, non-selective herbicides, particularly *N*-(phosphono- methyl) glycine type herbicides, such as Glyphosate and Sulfosate and phosphinyl amino acids, such as Glufosinate, particularly as the ammonium salt. Such water soluble actives can be used as the sole active in for example in aqueous solutions or in water dispersible granules, but more usually, they will be used in combination with water insoluble or immiscible actives in multi active formulations. In particular, formulations can be made up using a water soluble (non-specific) herbicide such as Glyphosate, Sulfosate and/or Glufosinate, with a selective herbicide, such as a sulphonyl urea e.g. metsulfuron-methyl, pyridine carboxylic acid e.g. clopyralid, aryloxy alkanoic acids e.g. 2,4-D, substituted ureas e.g. diuron, or 2-(4-aryloxyphenoxy)propionic acids e.g. clodinafoppropargyl, and/or with an insectcide and/or fungicide.

Generally, when used as adjuvants in agrochemical formulations, the compounds of and used in this invention can be added to agrochemical formulations as part of the tank mix (the formulation actually used for spraying) or can be included in pre-formulated products which usually take the form of concentrates, emulsifiable concentrates or solid dispersible granules.

When added to tank mix compositions for spray formulations using current spray application rates, generally from 100 to 400 l(spray).ha⁻¹ (crop treated), usually about 300 l.ha⁻¹, the concentration of the active agrochemical is typically from about 0.05 to about 3%, more usually from 0.1 to about 0.5 and particularly about 0.2 % by weight of the spray formulation and the concentration of adjuvant will typically be 0.02 to about 2%, more usually 0.2 to about 1% and particularly about 0.1 %. The weight ratio of active agrochemical to adjuvant is usually from 1:5 to 10:1, more usually from 1:2 to about 4:1. These figures correspond to crop application rates of the active agrochemical generally in the range 300 to 4000 g.ha⁻¹, more usually from 750 to about 2000 g.ha⁻¹ (the actual amount depending on the particular crop, agrochemical and effect desired). For low volume spraying, generally higher spray concentrations will be used, but the ratio of agrochemical to adjuvant will be within the ranges given above.

The surfactants of the formula (I) can be used as "built in" adjuvants in concentrate agrochemical formulations that are intended for dilution prior to use. In such concentrates, the concentration of active agrochemical is typically from about 5 to about 60%, more usually from 10 to 40% and the adjuvant concentration is from about 3 to about 50%, more usually from 5 to 30% by weight of the concentrate. The use as built in adjuvants in concentrates is particularly applicable for concentrates where the carrier is aqueous and the active is or includes one or more water soluble herbicides, such as Glyphosate, Sulfosate and Glufosinate.

As adjuvants the compounds of and used in this invention can provide faster effectiveness of agrochemicals especially water soluble herbicides, particularly of the glyphosate type, and can have significantly lower toxicity, particularly aquatic toxicity, than conventional adjuvants, particularly those based on fatty amine ethoxylates. The improved toxicity is also important when the compounds are used to provide other surfactant effects in agrochemical formulations.

When used as emulsifiers, dispersants, thickeners or solubilisers, the surfactants will usually be incorporated into concentrate forms of agrochemical formulation. The functions of the surfactants and the amounts typically used are:
Emulsifiers - emulsifier surfactants are included in concentrate formulation for diluting to make emulsions often, and desirably, as emulsifyable concentrates (concentrates including agrochemical active, either liquid or in solution in an organic liquid, and emulsifier which emulsifies spontaneously or with minimal stirring on dilution in water). The proportion of emulsifier surfactant is typically from 1 to 40%, commonly from 1 to 30%, more typically 3 to 15% by weight of the concentrated formulation, typically from 1 to 80% by weight, more usually from 3 to 50% by weight based on the total weight of the oil phase in the formulation (or formed when the formulation is diluted to form an emulsion);
Dispersants - dispersant surfactant is used to make solids dispersed in liquid carriers in concentrates more stable to settling or flocculation of the solids. The amount of surfactant used in typically from 1 to 30% by weight of the dispersed phase of the formulation;
Thickeners - surfactants can be used as thickeners or rheology modifiers in liquid concentrate formulations, especially in emulsion or emulsifyable concentrate formulations, to stabilise the concentrate formulation against settling flocculation or phase separation prior to dilution. The amount of surfactant used in typically from 0.01 to 5% by weight of the formulation and usually from 0.1 to 5% by weight of the non-aqueous, usually oil, phase in the formulation (or formed when the formulation is diluted to form an emulsion);
Solubilisers - surfactant solubilisers are typically used to increase the (mutual) solubility, miscibility or compatibility of other formulation components with the beneficial effect of increasing the stability of liquid, especially concentrate, formulations. The amount of surfactant used in typically from 10 to 40% by weight of the concentrate formulation, and possibly up to 80% by weight of the non-aqueous, usually oil, phase in the formulation (or formed when the formulation is diluted to form an emulsion).

When used as wetting agents i.e. principally to improve the wetting of plant leaves by the spray droplets, the surfactant can be included in a concentrate or added as a tank mix additive. The amount used will typically be from 0.0001 to 0.5%, more usually not more than about 0.1%, by weight of the (dilute) spray formulation and may be from 1 to 15% by weight of a concentrate.

Agrochemical formulations of the invention can be made up using surfactants of the formula (I) in a variety of formulation types including:
i Water soluble liquids (aqueous dilutable solutions) in which water soluble agrochemical active(s) and surfactant(s) are dissolved in water and the formulation is diluted with water before use. In such formulations the surfactant(s) are usually present as adjuvants or wetting agents. Typically such formulations use concentrations within the ranges:

| | |
|---|---|
| agrochemical active | 100 to 500 g.l⁻¹ |
| surfactant | 30 to 500 g.l⁻¹ |

The surfactant can be a mixture of compounds of the formula (I) and other, particularly non-ionic surfactants (see also below about mixtures).
Possible other components in such formulations include
i antifoams, particularly polysiloxane antifoams, typically included at a concentration of from 0.1 and 10% by weight of the concentrate formulation; and
ii viscosity modifiers : gums, e.g. xanthan gums, modified cellulose e.g. carboxy- methyl, - ethyl or -propyl cellulose, typically included at between 0.01 and 5% by weight of the concentrate formulation.

Such concentrate formulations can be made by simple mixing of the components.
Conveniently this may be carried out by dissolving the agrochemical active(s) and the adjuvant surfactant(s) and any other components in water to give either a concentrate for subsequent dilution to end use concentrations or directly at end use concentration e.g. in the spray tank.
Liquid concentrates, particularly emulsifiable concentrates, can include compounds of the formula (I). In liquid concentrates the surfactants are typically present as adjuvants, wetting agents, emulsifiers or solubilisers. The amount of surfactant(s) used in such concentrates is typically from 1 to 30% by weight of the concentrate. Other surfactants such as non-ionic, amphoteric, cationic or anionic or combinations of such surfactants may be used together with compounds of the formula (I) (see also below about mixtures). In liquid concentrates, typically use concentrations are within the ranges:

| | |
|---|---|
| agrochemical active | 0.2 to 10% by weight (though with liquid agrochemicals, the concentration can be up to 90%); and |
| surfactant | 1 to 20% by weight of the liquid concentrate. |

Liquid concentrate agrochemical formulations may also include:
solvents such as monoethylene glycol, diethylene glycol, glycerol, (mono)propylene glycol, which, especially with propylene glycol, may also act as a humectant, typically in an amount from 5 to 500% by weight of the surfactants;
oils, particularly vegetable or mineral oils, such as spray oils, typically in an amount from 5 to 500% by weight of the surfactants;
salts, such as ammonium chloride and/or sodium benzoate, and/or urea as gel inhibition aids typically in an amount from 1 to 10% by weight of the formulation.

Solid dispersible granules - the surfactant will usually be included as an adjuvant or a dispersing agent and can be included in a granular agrochemical active formulation or itself be formulated as dispersible granules. Typically granules including agrochemical active contain from 1 to 80%, more usually from 1 to 30%, by weight of the granule of active. When included in granules containing an agrochemical active, the adjuvant typically forms from 5 to 50% by weight of the granule.
The granules can include clathrates, particularly urea clathrates, in particular incorporating the surfactant, especially as an adjuvant. Such clathrates can be made by forming a co-melt, including the urea and surfactant, and cooling by e.g. spray cooling. Such clathrate solid granules will typically have a ratio of urea to surfactant adjuvant of from 1:2 to 5:1 by weight. Clathrates can be included in the agrochemical granules or and desirably formulated as a separate adjuvant granule which can be used by direct mixing with granular agrochemical active compositions.
When the adjuvant is provided in separate granules from the active agrochemical, the mixing rate of adjuvant granules to agrochemical active granules will depend on the respective concentrations in the granules, but will usually be such as to give a ratio of adjuvant to agrochemical active within the ranges described above.
In such granular formulations, other possible components of the granules include:
binders, particularly binders which are readily water soluble to give low viscosity solutions at high binder concentrations, such as polyvinylpyrrolidone, polyvinylalcohol, carboxymethyl cellulose, gum arabic, sugars, starch, sucrose and alginates;
diluents, absorbents or carriers such as carbon black, talc, diatomaceous earth, kaolin, aluminium, calcium and/or magnesium stearate, sodium tripolyphosphate, sodium tetraborate, sodium sulphate, sodium, aluminium or mixed sodium-aluminium silicates; and sodium benzoate;
disintegration agents, such as surfactants, materials that swell in water, for example carboxymethyl cellulose, collodion, polyvinyl pyrrolidone and/or microcrystalline cellulose swelling agents; salts such as sodium and/or potassium acetate, sodium carbonate, bicarbonate and/or sesquicarbonate, ammonium sulphate and/or dipotassium hydrogen phosphate;
wetting agents such as alcohol alkoxylates, particularly ethoxylates or ethoxylate/propoxylates;
dispersants such as sulphonated naphthalene formaldehyde condensates and acrylic copolymers; and
antifoam agents, typically at a concentration of from 1 to 10 % by weight of the granule.

Spray formulations at application concentration, including surfactants of the formula (1), particularly as adjuvants, can be made up by diluting/dispersing the agrochemical active and the adjuvant in the spray liquid (usually water). Also concentrate forms of the agrochemical formulation can be used, for example:
i liquid concentrate containing the agrochemical active and, particularly adjuvant, surfactant dissolved in water;
ii liquid concentrate containing the agrochemical active dissolved or dispersed in a non-aqueous, water immiscible liquid, which may be an emulsifiable concentrate and may include a proportion of water, including an adjuvant surfactant;
iii liquid concentrate containing the agrochemical active dissolved or dispersed in a non-aqueous, water miscible liquid and including an adjuvant surfactant;
iv a solid granular concentrate of or containing the agrochemical active and optionally including an adjuvant surfactant, or the adjuvant surfactant can be provided separately for example as a solution in a solvent (water or a non-aqueous solvent) or a granule, particularly a urea adduct, containing the adjuvant.

Concentrated forms of the agrochemical active will typically be diluted from 10 to 10000, particularly 30 to 1000 times to generate the agrochemical spray for use.

Agrochemical formulations often include more than one surfactant either because surfactants are used in combination to achieve the desired effect or used to provide different effects. It is thus possible in this invention to use combinations of more than one surfactant of the formula (I) or to combine surfactant(s) of the formula (I) with other surfactants.

For adjuvancy, mixtures of adjuvant surfactants can be used and the invention includes agrochemical formulations including compounds of the formula (I) in combination with other adjuvant materials. Commonly such other adjuvants may be non-ionic surfactant adjuvants and examples include so-called hydrocarbyl, particularly alkyl, polysaccharides (generally more correctly described as oligosaccharides); hydrocarbyl, particularly alkyl, amine alkoxylates, particularly ethoxylates, linear or mono-branched alcohol alkoxylates, particularly ethoxylates; sorbitol fatty acid esters; sorbitan fatty acid esters; and ethoxylated sorbitan fatty acid esters. The proportion of compounds of the formula (I) and other adjuvants, particularly non-ionic surfactant adjuvant, (when used) is typically from 1:5 to 10: 1, more usually from 1:1 to 5:1 by weight. The proportions and concentrations of adjuvants referred to above include both compound(s) of the formula (I) and other, particularly non-ionic surfactant adjuvants. Co-adjuvants, including ionic and/or inorganic materials, for example ammonium sulphate, may be included in adjuvant containing agrochemical formulations of the invention, particularly with non-ionic surfactant adjuvants, especially including those of the formula (I), optionally used in combination with other, particularly non-ionic, surfactant adjuvants.

Especially where emulsification is desired the surfactant(s) will usually be included in or with the formulation components including the phase to be emulsified. Other surfactants, especially non-ionic surfactants can be used together with the compounds of the formula (I).

Generally when other surfactants, especially non-ionic surfactants are used, the compound(s) of the formula (I) will be at least 25% and more usually at least 50% of the total surfactant used to provide the desired effect.

Other conventional components can be included in such formulations such as one or more oils e.g. mineral oil(s), vegetable oil(s) and alkylated vegetable oil(s) which are, typically C₁ to C₈, alkyl mono esters of vegetable oil fatty acids; solvents and/or diluents such as ethylene and/or propylene glycol or low molecular weight alcohols, which act to solubilise the formulation and/or to reduce the viscosity and/or to avoid or reduce dilution problems e.g. the formation of gels. In particular where non-aqueous, particularly those which are not miscible with or soluble in water, liquids are included e.g. as solvents for the agrochemical and/or in a concentrate to form an emulsion on dilution with water for spraying, other surfactants may be included as solubilisers and/or emulsifiers. Such materials will typically be chosen from anionic, cationic and/or non-ionic surfactants for their effectiveness in solubilisation and or emulsification. Such other surfactant components will, as with formulations using purely conventional surfactants, be used in amounts based on the desired effect.

Other surfactants may also be included to improve wetting. Examples of such wetting agents include nonionic surfactants such as alcohol ethoxylates for example of C₉ to C₁₅, particularly primary, alcohols, which may be linear or branched, particularly mono-branched, with from 5 to 30 moles of ethylene oxide; and alkoxylates of such alcohols particularly mixed ethoxylate/propoxylates which may be block or random mixed alkoxylates, typically containing from 3 to 10 ethylene oxide residues and from 1 to 5 propylene oxide residues, particularly where the polyalkoxylate chain is terminated with propylene oxide unit(s);
polyoxyethylene/polyoxypropylene copolymers, particularly block copolymers, such as the Synperonic PE series of copolymers available from Uniqema, and alkyl polysaccharides; anionic surfactants e.g. isethionates, such as sodium cocoyl isethionate, naphthalene sulphonic acids or sulphosuccinates. The amounts of wetting surfactants are typically similar to or the same as the levels typically used to provide adjuvant effects (see above).

The compounds of the formula (I) may be used in combination with non-surfactant materials, particularly solvents or solvation aids such as glycols such as monopropylene glycol and/or polyethylene glycol. The proportion of compounds of the formula (I) to such solvents or solvation aids, (when used) is typically from 1:5 to 10:1, more usually from 1:1 to 5:1 by weight.

The invention includes a method of treating vegetation by applying to plants and/or soil a composition including a surfactant of the formula (I) and an agrochemical according to the invention. The agrochemical may be one or more of the types of actives described above, particularly, one or more growth regulators, herbicides, and/or pesticides, for example insecticides, fungicides or acaricides. This method of the invention includes:
(i) a method of killing or inhibiting vegetation by applying a formulation which includes one or more growth regulators and/or herbicides and at least one compound of the general formula (I) as an adjuvant, and/or
(ii) a method of killing or inhibiting plant pests by applying a formulation which includes one or more pesticides, for example insecticides, fungicides or acaricides, and at least one compound of the general formula (I) as an adjuvant.

Other additives can be included in agrochemical formulations of the invention including:
inorganic salts such as ammonium chloride, calcium chloride and/or sodium benzoate and/or urea in an amount of from 0.01 to 1% by weight of composition.
antifoams which can be silicon based materials such as organopolysiloxanes, which are typically used in an amount from 0.1 to 10%, preferably 0.2 to 6% by weight of the surfactant; 0.01 to 5%, particularly 0.02 to 2% by weight of agrochemical concentrate and 0.0001 to 0.1 % preferably 0.001 to 0.05% by weight of a spray formulation at end use dilution;
viscosity modifiers, particularly gums such as xanthan gums; cellulose derivatives, such as carboxyl-methyl, -ethyl, or -propyl cellulose, typically used at from 0.01 to 5 wt % of a concentrated formulation; and
other non surfactant materials such as stabilisers and/or anti-microbials, typically used at from 0.01 to 5 wt % of a concentrated formulation.

The following Examples illustrate the invention. All parts and percentages are by weight unless otherwise stated.

| Materials | |
|---|---|
| N-methylglucamine | *N*-methyl-*N*-( 1-deoxyglucityl)amine |
| bis-sorbitylamine | bis(1 1-deoxyglucit-1-yl)amine |
| dodecyl-poly-4-oxyethylene | Brij 30 *ex* Uniqema |
| tridecyloxy poly-5.7-oxyethylene | Cresmer PTCD *ex* Uniqema (India) |
| Sulfosate | glyphosate trimethylsulphonium salt as a solution in water containing 720 g.l⁻¹ active salt |
| Glyphosate | glyphosate *iso*-propylamine salt as a solution in water containing 767 g.l⁻¹ active salt |
| Horizon EW | tebuconazole 250 g.l⁻¹ active material *ex* Bayer |
| Roundup-ultra | commercially available glyphosate formulation *ex* Monsanto |
| MON0818 | tallow amine (20) ethoxylate ex Monsanto |
| T150 | Genamin T150 - tallow amine (15) ethoxylate *ex* Clariant |

Synthesis Examples SE1 to SE26 illustrate the synthesis of the compounds of the formula (I).

### Synthesis Example SE1 : N-(1-deoxyglucity)N-(2-hydroxydodecyl)-N-methylamine

N-methylglucamine (4.8 g; 24.6 mmol) was reacted with 1-epoxydodecane (5 g; 27.2 mmol) in propylene glycol (2.45 ml) at 120°C for 3 hours. The product was initially a highly viscous transparent liquid, solidified after keeping at ambient temperature overnight. The identity of the product was verified using IR and NMR spectroscopy.

The following further compounds were made by the general method of SE1 substituting the corresponding starting material for the 1-epoxydodecane used in SE1.
SE2 *N*-(2-hydroxy(mixed hexadecyl/octadecyl)amino-1-deoxyglucitol
SE3 *N*-(2-hydroxytetradecylamino)-1-deoxyglucitol
The identity of the products was verified using IR and NMR spectroscopy.

### Synthesis Example SE4 : NN-bis(1-deoxyglucityl)-N-(2-hydroxydodecyl)-amine

SE1 was repeated except that bis-sorbitylamine was used instead of the N-methyl glucamine used in SE1. The product solidified after cooling. The identity of the product was verified using IR and NMR spectroscopy.

The following further compound was made by the general method of SE4 substituting the corresponding starting material for the 1-epoxydodecane used in SE4.
SE5 *NN*-bis(1-deoxyglucityl)-*N*-(2-hydroxytetradecyl)-amine
The identity of the product was verified using IR and NMR spectroscopy.

### Synthesis Example SE6 : 1-(N-methyl-N-1-deoxyglucityl)amino-2-hydroxy-3-dodecyloxy propane

*N*-Methylglucamine (4.03 g; 20.7 mmol) was reacted with dodecyl glycidyl ether (5 g; 20.7 mmol) at 120 to 130°C for 2 to 3 hours and the mixture was then cooled. The product was a colourless highly viscous liquid which turned to a white solid on keeping overnight. The identity of the product was verified using IR and NMR spectroscopy.

The following further compounds were made by the general method of SE6 substituting the corresponding starting material for the dodecyl glycidyl ether used in SE6.
- SE7: 1-(N-methyl-N-1-deoxyglucitylamino)-2-hydroxy-3-octyloxy propane
- SE8: 1-(*N*-methyl-*N*-1-deoxyglucitylamino)-2-hydroxy-3-(2-ethylhexyl)oxy propane
- SE9: 1-(N-methyl-*N*-1-deoxyglucitylamino )-2-hydroxy-3-nonyloxy propane
- SE10: 1-(*N*-methy)-*N*-1-deoxyglucitylamino)-2-hydroxy-3-decyloxy propane
- SE11: 1-(*N*-methyl-*N*-1-deoxyglucitylamino)-2-hydroxy-3-(mixed octyloxy/decyloxy) propane
- SE12: 1-(*N*-methyl-*N*-1-deoxyglucitylamino)-2-hydroxy-3-tetradecyloxy propane
- SE13: 1-(*N*-methyl)-*N*-1-deoxyglucitylamino)-2-hydroxy-3-(mixed dodecyloxy/tetradecyloxy) propane
- SE14: 1-(*N*-methyl-*N*-1-deoxyglucitylamino)-2-hydroxy-3-(branched undecyloxy) propane
The identity of these was verified using IR and NMR spectroscopy.

### Synthesis Example SE15 : 1-NN-bis(1-deoxyglucityl)amino)-2-hydroxy-3-dodecyloxy propane

SE6 was repeated except that bis-sorbitylamine was substituted for the *N*-methylglucamine used in SE6. The identity of the product was verified using IR and NMR spectroscopy.

The following further compounds were made by the general method of SE15 substituting the corresponding starting material for the dodecyl glycidyl ether used in SE15.
- SE16: 1-(N-bis-sorbitylamino)-2-hydroxy-3-(mixed dodecyloxy/tetradecyloxy) propane
- SE17: 1-(*N*-bis-sorbitylamino)-2-hydroxyl-3-tetradecyloxy propane
The identity of the products was verified using IR and NMR spectroscopy.

### Synthesis Example SE18 : 1-(N-methyl-N-1-deoxyglucitylamino)-2-hydroxy]-3-(dodecyloxy-poly-4-oxyethyleneoxy)propane

N-Methylglucamine (0.37 g; 1.91 mmol) was reacted with dodecyloxy-poly-4-oxyethylene glycidyl ether (1 g; 2.54 mmol) at 125 to 130°C for 2 to 3 hours. The product was obtained as a transparent viscous liquid. The identity of the product was verified using IR and NMR spectroscopy.

The following further compounds were made by the general method of SE18 but substituting the corresponding starting material for the dodecyl glycidyl ether and/or N-methylglucamine used in SE18.
- SE19: 1-(*N*-methyl-*N*-1-deoxyglucitylaminohydroxyl-3-(tridecyloxy poly-5.7-oxyethyleneoxy) propane
- SE20: 1-(*N*-bis-sorbitylamino)-2-hydroxyl-3-tetradecyloxy propane
- SE21: 1-(*N*-1-deoxyglucitylamino)-2-hydroxyl-3-(branched octadecyloxy poly-10PO-10EO) propane*
- SE22: 1-(*N*-methyl-*N*-1-deoxyglucitylamino)-2-hydroxyl-3-(branched octadecyloxy poly-4PO-10EO) propane*
The identity of these products was verified using IR and NMR spectroscopy.
* The precursor used was a branched C₁₈ alcohol 10-propoxylate-10-ethoxylate glycidyl ether itself made by reacting a monobranched C₁₈ alcohol 10-propoxylate-10-ethoxylate with *epi*-chlorohydrin.

### Synthesis Example SE23 : Betaine from 1-(N-1-deoxyglucitylamino)-2-hydroxy-3-dodecyloxy propane

The direct reaction product of the heating stage from a repeat of Example SE6, using 66.9 g (343 mmol) of N-methylglucamine and 83.05 g (343 mmol) of dodecyl glycidyl ether), was cooled to 90 to 95°C, water (100 ml) was added and an aqueous solution of sodium chloroacetate (40.14 g; 343 mmol, dissolved in 90 ml water) was added slowly to this stirred mixture keeping the temperature at 90 to 95° C. The resultant mixture was further stirred at 90 to 95°C for 1 hour to give the title compound product as a clear colourless liquid (50% active in water).

The following further compound was made by the general method of SE23 but substituting 1-(*N*-methyl-*N*-1-deoxyglucitylamino)tetradecyl glycidyl ether for the corresponding dodecyl glycidyl ether used in SE23.

### SE24 Betaine from 1-(N-Methylglucamino)-2-hydroxy-3-tetradecyloxy propane

### Synthesis Example SE25 : 1-(N,N-bis(N-1-deoxyglucitylamino)amonium)-2-hydroxy-3-dodecyloxy propane sulphate

The direct reaction product of the heating stage from a repeat of Example SE15, using 149.2 g (433 mmol) of N-methylglucamine and 104.8 g (433 mmol) of dodecyl glycidyl ether, was cooled to 40 to 45°C, isopropyl alcohol (63 ml) added and the mixture stirred. Dimethyl sulphate (49.1 g; 390 mmol) was added to the stirred mixture over a period of 4 to 5 minutes and the resultant mixture stirred for 1 hour at which time the acid value was 9.6. Isopropyl alcohol was then removed under reduced pressure and water (26.5 ml) added to give the product as a white viscous liquid (90% active in water).

The following further compound was made by the general method of SE25 but substituting 1-(*N*-methyl-*N*-1-deoxyglucitylamino)tetradecyl glycidyl ether for the corresponding dodecyl glycidyl ether used in SE25.

### SE26 1-(NN-dimethyt-N-1-deoxyglucitylamino)-2-hydroxy-3-tetradecyloxy propane sulphate

The products of some of the synthesis examples (if necessary after purification) were tested for aquatic toxicity to *Daphnia magna* in a standard bio-assay to derive the EC50 (in mg.l⁻¹) for immobilising the Daphnia at the end of the 48 hour assay procedure. The results are given below:

| Compound (SE No) | EC50 |
|---|---|
| SE20 | >100 |
| SE21 | 31.4 |
| MON0818* | 2.0 |

| | |
|---|---|
| * conventional fatty amine ethoxylate adjuvant particularly used with glyphosate type hergbicides - toxicity data from 'Glyphosate: A unique global herbicide' by J E Franz *et al,* ACS Monograph 189, 1997 (96 hour figure). | |

These data indicate that the compounds of the formula (I) have significantly lower aquatic toxicity as compared with the conventional tallow amine ethoxylate adjuvant.

The irritancy of the products of some of the synthesis examples (if necessary after purification) was tested using standard testing protocols. The results are given below:

| Compound (SE No) | Irritation assessment | |
|---|---|---|
| | Skin | Eye |
| FAE* | severe | severe |
| SE10 | practically none | moderate/severe |
| SE23 | slight | moderate |
| SE25 | slight | moderate |
| SE21 | slight/moderate | practically none/slight |

| | | |
|---|---|---|
| * fatty amine ethoxylate (previously obtained data) | | |

These data indicate that the compounds of and used in this invention are substantially less irritant than tallow amine ethoxylates such as are conventionally used as agrochemical adjuvants

Application Examples AE1 to AE5 illustrate the application of compounds of the formula (I) as agrochemical adjuvants. For convenience, most of the adjuvants used in these Examples were diluted with monopropyleneglycol (MPG). The formulations, referenced as SE..M, used were:

| SEM No | SE No | % amine | % MPG |
|---|---|---|---|
| SE3M | SE3 | 50 | 50 |
| SE5M | SE5 | 50 | 50 |
| SE10M | SE10 | 100 | 0 |
| SE12M | SE12 | 50 | 50 |
| SE14M | SE14 | 75 | 25 |
| SE17M | SE17 | 50 | 50 |
| SE22M | SE22 | 65 | 35 |
| SE24M | SE24 | 40 | 60 |
| SE26M | SE26 | 70 | 30 |

For some of the synthesised materials, reaction was not complete so the percentage figure for "amine" may overstate the actual proportion of amine in the additive as used.

In some of the Application Examples crop species are used for testing herbicidal effectiveness. This was done because crop plants do tum up as weeds (in other crops), they can be good models for effectiveness on certain types of weed and they are available as controlled seeds thus improving consistency in testing (weed seeds are much less readily available in such controlled forms).

### Application Example AE1

Aqueous herbicide formulations were made up using Sulfosate as the active material at 3.3 g.l⁻¹ and (diluted) adjuvant at 2.4 g.l⁻¹. The formulations were tested for herbicidal activity on species of *Hordeum vulgare ssp.* (barley), *Lolium multiflorum* (Italian rye grass) and *Pisum sativum* (pea) by spraying the plants with 300 l.ha⁻¹ (equivalent to 990 g(active).ha⁻¹) herbicide formulation.
Chlorosis and/or necrosis and growth reduction were assessed after 6, 10 and 16 days with the results quoted as a percentage of the plants so affected. The results are set out in Table 1 below with Chlorosis and /or necrosis and growth reduction data separated by a colon.

**Table 1**

| Ex No | Adjuvant | days | Species | | |
|---|---|---|---|---|---|
| | | | barley | rye grass | pea |
| AE1.1 | SE3M | 6 | 90:50 | -:65 | 60:65 |
| | | 10 | 100:75 | -:75 | 65:65 |
| | | 16 | 100:80 | -:95 | 75:80 |
| AE1.2 | SE5M | 6 | 65:40 | -:45 | 25:30 |
| | | 10 | 98:50 | -: 75 | 10:50 |
| | | 16 | 100:80 | -:95 | 15:60 |
| AE1.3 | SE12M | 6 | 65:40 | -:45 | 55:50 |
| | | 10 | 98:50 | :75 | 60:65 |
| | | 16 | 100:80 | :95 | 65:75 |
| AE1.4 | SE16M | 6 | 65:40 | -:45 | 25:20 |
| | | 10 | 98 : 50 | -:75 | 10 : 40 |
| | | 16 | 100:80 | -:95 | 10:60 |

### Application Example AE2

A field trial was carried out using Sulfosate as active herbicide to investigate the effectiveness of adjuvants of the invention in weed control. The herbicide formulations were aqueous solutions of the herbicide (3.3 g.l⁻¹) and adjuvant (1.65 g.l⁻¹) in water which was applied by spraying at an application rate of 300 l.ha⁻¹, equivalent to 990 g.ha⁻¹ of active Sulfosate salt and 495 g.ha⁻¹ of adjuvant. An untreated control was used as the basis for comparison and a control treatment with Sulfosate without adjuvant was also included. Weed control was assessed by visual observation on a scale of 0 = 'no effect' to 100 = 'all weeds killed' at 7, 14 and 21 days after spraying. 4 replications of 2 m x 8 m plots containing mixtures of the following weeds were used:

| Weed | Growth stage | Weed | Growth stage |
|---|---|---|---|
| *Chenopodium album* | 4-6 leaves | *Galinsoga parviflora* | 2-4 leaves |
| *Polygonum persicaria* | 4-5 leaves | *Solanum nigra* | 2-4 leaves |
| *Stellaria media* | 4-6 leaves | *Poa annua* | 3-4 tillages |
| *Urtica urens* | 4 leaves | | |

The weed control results are set out in Table 2 below.

**Table 2**

| Ex No | Adjuvant type | % weed control | | |
|---|---|---|---|---|
| | | 7 days | 14 days | 21 days |
| AE2.1C | - | 25 | 50 | 45 |
| AE2.1 | SE5M | 37.5 | 67.5 | 60 |
| AE2.2 | SE3M | 60 | 85 | 80 |
| AE2.3 | SE16M | 47.5 | 77.5 | 70 |
| AE2.4 | SE12M | 60 | 82.5 | 80 |

### Application Example AE3

A further field trial was carried out generally as described in AE2, but using Glyphosate as active herbicide. The same plot set up, selection of weeds and assessment was used as in AE2. The effective application rate of the Glyphosate was 1080 g.ha⁻¹ and of the adjuvant 540 g.ha⁻¹. The results are set out in Table 3 below.

**Table 3**

| Ex No | Adjuvant type | % weed control | | |
|---|---|---|---|---|
| | | 7 days | 14 days | 21 days |
| AE2.1 C | - | 35 | 65 | 65 |
| AE2.1 | SE5M | 35 | 65 | 65 |
| AE2.2 | SE3M | 47.5 | 72.5 | 77.5 |
| AE2.3 | SE16M | 47.5 | 72.5 | 65 |
| AE2.4 | SE12M | 50 | 72.5 | 80 |

### Application Example AE4

A multi crop trial was run with Glyphosate as herbicide. The selected crops: Italian ryegrass (*Lolium muftiflorum*)*,* Pea (*Pisum sativum*) and Savoy cabbage (*Brassica oleracea* var. *rapa*) were sown on a sandy lawn soil in strips with 40 m long and 2 m wide. Glyphosate and adjuvant combinations were applied across the crop strips in 2 m wide bands (one replicate), sprayed at 250 l.ha⁻¹. Glyphosate at 1080 g(active).ha⁻¹ + 540 g.ha⁻¹ adjuvant was sprayed. An untreated control was carried out. Visual evaluation of crop growth reduction and chlorosis/necrosis were made of the different crops 7, 10 and 16 days after treatment. Each parameter was estimated as percentage as compares with nearest untreated control plot (= 0%). The results are set out in Table 4 below.

**Table 4**

| Ex No | Compound | days | Species | | |
|---|---|---|---|---|---|
| | | | rye grass | pea | cabbage |
| AE4.1 | SE5M | 7 | 45:45 | 25:25 | 40:82 |
| | SE3M | 10 | 90:75 | 5:45 | 70:85 |
| | SE16M | 16 | 100:95 | 5:55 | - 88 |
| AE4.2 | SE12M | 7 | 45: 50 | 35:35 | 45:85 |
| | SE5M | 10 | 90: 75 | 10:50 | 75 85 |
| | SE3M | 16 | 100:95 | 10:55 | -:93 |
| AE4.3 | SE16M | 7 | 35:40 | 30:25 | 35:85 |
| | SE12M | 10 | 85: 75 | 5:45 | 75:88 |
| | SE5M | 16 | 100: 95 | 5:50 | - :92 |
| AE4.4 | SE3M | 7 | 45:50 | 40:40 | 95:95 |
| | SE16M | 10 | 92:75 | 15:50 | 95:95 |
| | SE12M | 16 | 100: 95 | 20:70 | - :98 |

### Application Example 5

Field trials were carried out to test the effectiveness of fungicide on winter wheat (variety Versailles) using the active Horizon EW (tebuconazole) (250 g.l⁻¹) in the control of fungal leaf rust (*Puccinia recondita*). The plots were sprayed at a spray volume 300 l.ha⁻¹, with the adjuvants added as tank mix additives at a concentration of 0.1% weight/volume on the spray. The normal application rate (NAR) for Horizon alone is 1 l.ha⁻¹ (250 g.ha⁻¹) and this was used as a control together with 0.75 l.ha⁻¹ (187.5 g.ha⁻¹; 3/4 NAR). An untreated control was also included. For these trials, adjuvant containing formulations were at 3/4 NAR for the active and 0.1% weight/volume (300 g.ha⁻¹) adjuvant. The effect of the spraying was assessed 3 weeks after treatment and is expressed as % infected leaf area (2 and 3 top leaves).

**Table 5**

| Ex No | Fungicide | | Adjuvant | | % infected | |
|---|---|---|---|---|---|---|
| | type | g.ha⁻¹ | type | g.ha⁻¹ | 2 leaf | 3 leaf |
| AE5.1 C | tebuconazole | 250 | - | - | 2.5 | 2.5 |
| AE5.2C | tebuconazole | 187.5 | - | - | 2.5 | 10 |
| AE5.1 | tebuconazole | 250 | SE5M | 300 | 0 | 0 |
| AE5.2 | tebuconazole | 187.5 | SE3M | 300 | 0 | 0 |
| AE5.3 | tebuconazole | 250 | SE16M | 300 | 0 | 2.5 |
| AE5.4 | tebuconazole | 187.5 | SE12M | 300 | 0 | 0 |

### Application Example AE6

Field trials were carried out on multi-crop test plots to test the effectiveness of compounds of the formula (I) as adjuvants for Glyphosate herbicide. The herbicide used was aqueous Glyphosate applied at the same rate as used in AE3. Roundup-Ultra at an application rate of 1080 g(active Glyphosate).ha⁻¹ was used as a control. Three crop plants were used as the test species:

| | | |
|---|---|---|
| Crop | Scientific name | Crop stage at spraying |
| Savoy cabbage | *Brassica oleracea* var, *sabauda* | 3-4 leaves, 10-12 cm |
| Flax | *Linum usitatissimum* | 20-25 cm |
| Pea | *Pisum sativum* | 5-6 branches, 20-30 cm |

The effctiveness of the compositions was assessed by visual evaluation of the percentage crop growth reduction in comparison with control plots which were not sprayed with herbicide (0% growth reduction) at 7, 11, 16 days after treatment and also at 28 days for Pea as differences became more pronounced with time. The formulations using compounds of the formula (I) clearly have a high speed of action as compared to Roundup-ultra. For some plots an effect is noticeable after as tittle as 2 days.

**Table 6**

| Ex No | Adjuvant | Flax | | | Pea | | | | Savoy cabbage | | |
|---|---|---|---|---|---|---|---|---|---|---|---|
| | | *7d* | *11d* | *16d* | *7d* | *11d* | *16d* | *28d* | *7d* | *11d* | *16d* |
| AE6.1 | SE10M | 75 | 90 | 95 | 75 | 90 | 95 | 98 | 85 | 95 | 99 |
| AE6.2 | SE12M | 65 | 90 | 95 | 60 | 80 | 85 | 90 | 75 | 90 | 99 |
| AE6.3 | SE22M | 80 | 90 | 95 | 75 | 88 | 98 | 99 | 75 | 95 | 100 |
| AE6.4 | SE14M | 70 | 90 | 95 | 70 | 85 | 90 | 97 | 70 | 95 | 100 |
| AE6.5 | SE24M | 60 | 90 | 95 | 47 | 65 | 70 | 80 | 50 | 88 | 97 |
| AE6.6 | SE26M | 65 | 90 | 95 | 60 | 78 | 85 | 92 | 60 | 92 | 98 |
| AE6.1C | - | 50 | 88 | 95 | 53 | 75 | 88 | 95 | 55 | 90 | 97 |

### Application Example AE7

Greenhouse trials were carried out to investigate the effect of rain on the adjuvant performance of a variety of glucamine based surfactants as adjuvants. The test species used were pea and barley plants at the 4-5 leaf stage (4 replicates) using Glyphosate as the agrochemical applied by spraying at various application rates with a weight ratio of glyphosate to adjuvant of 2:1 and using a spray volume of 200l/ha⁻¹. Some tests substrates were sprayed with water after applying the herbicide formulations to simulate the effect of rain on the effectiveness of the herbicide. Then effectiveness of the herbicide formulations was assessed visually with results expressed as percentage kill 11 Days after application of the herbicide formulations. The results are set out in Table 7 below. The adjuvants are identified by their SE..M Nos and the comparison material used in control Example AE7.1 C was Roundup-ultra. These data indicate good herbicide performance with only modest reductions in efficacy for samples exposed to simulated rain (indicated '+').

**Table 7**

| Ex No | Adjuvant | Barley | | | | Pea | | | |
|---|---|---|---|---|---|---|---|---|---|
| | | Glyphosate dose | | | | Glyphosate dose | | | |
| | | 324 g.ha⁻¹ | | 756 g.ha⁻¹ | | 324 g.ha⁻¹ | | 756 g.ha⁻¹ | |
| | | Rain | | | | Rain | | | |
| | | - | + | - | + | - | + | - | + |
| AE7.1 | SE14 M | 29 | 21 | 57 | 36 | 50 | 29 | 54 | 39 |
| AE7.2 | SE26 M | 36 | 25 | 50 | 25 | 46 | 25 | 68 | 32 |
| AE7.3 | SE24M | 39 | 21 | 50 | 29 | 39 | 21 | 54 | 32 |
| AE7.1C | - | 36 | 21 | 43 | 29 | 54 | 29 | 68 | 50 |

### Application Example AE8

Field trial Herbicide tests using Glyphosate were run using a glucamine based adjuvants and T150 (amine ethoxylate) for comparison on three test crops: *Convulvulus arvensis,* Wheat and *Malva sylvestris*. The application rate of the active agrochemical was varied as was the ratio of agrochemical to adjuvant (2:1 and 2:0.25 - indicated as '1' and 0.25' in Table 8 below) to obtain some dose response data. The Results in table 8 below show that the adjuvants of this invention provided adjuvant effects broadly as good as the conventional amine ethoxylate.

**Table 8**

| Ex No | SEM No | Time | *Convulvulus arvensis* | | | | Wheat | | | | *Malva sylvestris* | | | |
|---|---|---|---|---|---|---|---|---|---|---|---|---|---|---|
| | | | 540 g.ha⁻¹ | | 1080 g.ha⁻¹ | | 350 g.ha⁻¹ | | 700 g.ha⁻¹ | | 350 g.ha⁻¹ | | 700 g.ha⁻¹ | |
| | | | 1 | 0.25 | 1 | 0.25 | 1 | 0.25 | 1 | 0.25 | 1 | 0.25 | 1 | 0.25 |
| AE8.1 | SE14 M | 6 | 18 | 14.3 | 14.3 | 18 | 23.1 | 20 | 25 | 21.4 | 14.3 | 18 | 21.4 | 21.4 |
| | | 14 | 14.3 | 18 | 39.3 | 39.3 | 57.1 | 39.3 | 71.4 | 57.1 | 21.4 | 32.1 | 46.4 | 43 |
| | | 20 | 17.9 | 17.9 | 46.4 | 42.9 | 60.7 | 35.7 | 89.3 | 67.9 | 35.7 | 28.6 | 46.4 | 39.3 |
| AE8.2 | SE22M | 6 | 21.4 | 18 | 14.3 | 14.3 | 21.4 | 20 | 21.4 | 20 | 14.3 | 21.4 | 21.4 | 29 |
| | | 14 | 14.3 | 18 | 43 | 32.1 | 60.7 | 35.7 | 71.4 | 75 | 35.7 | 39.3 | 60.7 | 43 |
| | | 20 | 17.9 | 25 | 67.9 | 42.9 | 75 | 35.7 | 89.3 | 89.3 | 28.6 | 32.1 | 57.1 | 46.4 |
| AE8.1C | - | 6 | 18 | 14.3 | 18 | 14.3 | 14.3 | 18 | 21.4 | 21.4 | 18 | 18 | 25 | 21.4 |
| | | 14 | 39.3 | 21.4 | 60.7 | 43 | 64.3 | 50 | 71.4 | 64.3 | 32.1 | 25 | 57.1 | 43 |
| | | 20 | 39.3 | 21.4 | 64.3 | 60.7 | 78.6 | 78.6 | 96.4 | 85.7 | 35.7 | 25 | 60.7 | 57.1 |

## Claims

1. An agrochemical composition which includes an agrochemically active compound and a compound of the formula (I):
R¹ - (R²) X¹ *-* [ Link ] - R³ (I)
where
R¹ is polyhydroxy hydrocarbyl;
R² is H or hydrocarbyl, or is a group as defined for R¹;
X¹ is N; N⁺->O⁻; N⁺R⁴ - where: R⁴ - is C₁ to C₆ hydrocarbyl carrying an anionic substituent, particularly - CH₂ - COO⁻; or N⁺R⁵ An⁻ where: R⁵ is a C₁ to C₂₀ hydrocarbyl; and An⁻ is a charge balancing anion;
Link is a linking group of the formula: - CH₂ - CHOH - X² -
where X² is
a direct bond; - CH₂ - O -;- CH₂ - N(R⁶) -;- CH₂ - (OA)ₚ- O -; or - CH₂ - (OA)ₚ- N(R⁷) -;
where
OA is an oxyalkylene residue;
p is from 1 to 100;
R⁶ is H; C₁ to C₈ hydrocarbyl; or a group R¹ - (R²)X¹ - CH₂- CHOH - CH₂ - where R¹, R² and X¹ are as defined above; and
R⁷ is H; C₁ to C₈ hydrocarbyl; or a group R¹ - (R²) X¹ - CH₂- CHOH - CH₂- (OA)ₚ- where R¹, R², X¹, OA and p are as defined above; and
R³ is hydrocarbyl.

2. A composition as claimed in claim 1 wherein R¹ is a polyhydroxy alkyl group having a linear C₄ to C₇ chain and at least three hydroxyl groups directly bonded to chain carbon atoms.

3. A composition as claimed in claim 2 wherein R¹ is a group of the formula:
- CH₂ - (CHOH)₄ - CH₂OH.

4. A composition as claimed in any one of claims 1 to 3 wherein R² is an alkyl, hydroxyalkyl or alkoxyalkyl group, R⁵ is an alkyl, hydroxyalkyl, alkoxyalkyl or aralkyl, An⁻ is and alkali metal or ammonium ion, R⁶ and R⁷ are each independently alkyl or alkenyl groups and R³ is a C₁₀ to C₃₀ alkyl, alkenyl, alkaryl, aryl or aralkyl group.

5. A composition as claimed in any one of claims 1 to 4 wherein the oxyalkylene group(s) OA is(are) oxyethylene, oxyproylene or mixtures of oxyethylene and oxypropylene groups and p is from 1 to 50.

6. A composition as claimed in any one of claims 1 to 5 wherein Link is a group of one of the formulae: - CH₂- CHOH - CH₂ - O -;- CH₂ - CHOH - CH₂ - (OA)ₚ- O - ;
- CH₂ - CHOH - CH₂ - N(R⁶)-; or - CH₂ - CHOH - CH₂ - (OA)ₚ- N(R⁷) -; where OA, p, R⁶ and R⁷ are as defined in either claim 1 or claim 2.

7. A composition as claimed in any one of claims 1 to 6 wherein the agrochemically active compound is one or more plant growth regulators, herbicides, and/or pesticides, for example insecticides, fungicides, acaricides, nematocides, miticides, rodenticides, bactericides, molluscicides and/or bird repellants.

8. A composition as claimed in claim 7 wherein the agrochemically active compound is or includes at least one water soluble herbicide.

9. A composition as claimed in claim 8 wherein the water soluble herbicide is or includes at least one phosphonomethyl glycine, particularly Glyphosate and/or Sulfosate; at least one phosphinyl amino acid, particularly Glufosinate; and/or at least on bipyridinium compound, particularly Paraquat.

10. A compound of the general formula (IIa): R¹-(R²)X¹-[Link¹]-R³ where X¹, R¹, R², and R³ are as defined in any one of claims 1 to 5 and Link¹ is a linking group of one of the formulae: - CH₂-CHOH-CH₂-(OA)ₚ-O-; -CH₂-CHOH-CH₂-N(R⁶)-; or -CH₂-CHOH-CH₂-(OA)ₚ-N(R⁷)-; where OA, p, R⁶ and R⁷ are as defined for formula (I) in any one of claims 1 to 5.

11. A compound as claimed in claim 10 wherein X¹ is N; Link¹ is of the formula: - CH₂-CHOH-CH₂-(OA)ₚ-O-; and R¹ is a polyhydroxy alkyl group having a linear C₄ to C₇ chain and at least three hydroxyl groups directly bonded to chain carbon atoms.

12. A compound as claimed in claim 11 wherein the group R¹ is a residue of a sugar, particularly a monosaccharide such as glucose, fructose or sorbitol, a disaccharide such as maltose or palitose or a higher oligosaccharide.

13. A compound of the general formula (IIb): R¹-(R²)X^{1a}-[Link²]-R³ where R¹, R², and R³ are as defined above for formula (I); X^{1a} is N⁺->O- , N⁺R⁴ -or R⁵An⁻ where: R⁴ -, R⁵ and An⁻ are as defined above for formula (I); and Link² is a linking group of one of the formulae: - CH₂-CHOH-CH₂-O-; -CH₂-CHOH-CH₂-(OA)ₚ-O- ; -CH₂-CHOH-CH₂-N(R⁶)-; or - CH₂-CHOH-CH₂-(OA)ₚ-N(R⁷)-; where OA, p, R⁶ and R⁷ are as defined for formula (I) in any one of claims 1 to 5.

14. A compositon as claimed in claim 1 wherein the compound of the formula (I) is a compound of the formula (IIa) as defined in clany one of claims 10 to 12 or a comound of the formula (IIb) as defined in claim 13.

15. A method of treating vegetation by applying to plants and/or soil a composition as claimed in any one of claims 1 to 9 or 14.

16. A method of killing or inhibiting vegetation by applying a formulation as claimed in claim 1 which includes one or more growth regulators and/or herbicides and at least one compound of the general formula (I) as defined in any one of claims 1 to 6 or of the formula (IIa) as defined in claim 10 or formula (IIb) as defined in claim 11, as an adjuvant.

17. A method of killing of plant pests by applying a formulation as claimed in claim 1 which includes one or more pesticides, for example insecticides, fungicides or acaricides, and at least one compound of the general formula (I) as defined in any one of claims 1 to 6, or of the formula (IIa) as defined in claim 10 or formula (IIb) as defined in claim 11, as an adjuvant.

18. The use of a compound of the formula (I) as defined in anyone of claims 1 to 6, or of the formula (IIa) as defined in claim 10 or formula (IIb) as defined in claim 11 as an agrochemical adjuvant.

19. The use as claimed in claim 18 wherein the agrochemically active compound is one or more plant growth regulators, herbicides, and/or pesticides, for example insecticides, fungicides, acaricides, nematocides, miticides, rodenticides, bactericides, molluscicides and/or bird repellants.

20. The use as claimed in claim 19 wherein the agrochemically active compound is or includes at least one water soluble herbicide.

21. The use as claimed in claim 20 wherein the agrochemically active compound is or includes at least one phosphonomethyl glycine, particularly Glyphosate and/or Sulfosate; at least one phosphinyl amino acid, particularly Glufosinate; and/or at least on bipyridinium compound, particularly Paraquat.

## Patentansprüche

1. Agrochemische Zusammensetzung, welche beinhaltet eine agrochemisch aktive Verbindung und eine Verbindung der Formel (I):
R¹- (R²) X¹- [Link] -R³ (I)
in der
R¹ Polyhydroxykohlenwasserstoff ist;
R² H oder Kohlenwasserstoff, oder eine wie für R¹ definierte Gruppe ist;
X¹ N ist; N+->O⁻; N⁺R⁴⁻ wobei: R⁴⁻ ist ein C₁- bis C₆-Kohlenwasserstoff tragend einen anionischen Substituenten, besonders - CH₂-COO⁻; oder N⁺R⁵An⁻ wobei: R⁵ ist ein C₁- bis C₂₀-Kolenwasserstoff; und An⁻ ist ein die Ladung ausgleichendes Anion;
Link ist eine verknüpfende Gruppe der Formel: - CH₂-CHOH-X²-, wobei X² ist
eine direkte Bindung; -CH₂-O-; -CH₂-N(R⁶) -; - CH₂-(OA)ₚ-O-; oder -CH₂-(OA)ₚ-N(R⁷) -;
wobei
OA ist ein Oxyalkylen-Rest;
p ist von 1 bis 100;
R⁶ ist H; C₁- bis C₈-Kohlenwasserstoff; oder eine Gruppe R¹-(R²)X¹-CH₂-CHOH-CH₂- wobei R¹, R² und X¹ wie oben definiert sind;
und
R⁷ ist H; C₁- bis C₈-Kohlenwasserstoff; oder eine Gruppe R¹- (R²) X¹-CH₂-CHOH-CH₂- (OA)ₚ- wobei R¹, R², X¹, OA und p wie oben definiert sind, und
R³ Kohlenwasserstoff ist.

2. Zusammensetzung nach Anspruch 1, wobei R¹ eine Polyhydroxyalkylgruppe mit einer linearen C₄- bis C₇-Kette und mindestens drei Hydroxygruppen, die direkt an die Kettenkohlenstoffatome gebunden sind, ist.

3. Zusammensetzung nach Anspruch 2, wobei R¹ ist eine Gruppe der Formel:
- CH₂- ( CHOH ) ₄-CH₂OH.

4. Zusammensetzung nach einem der Ansprüche 1 bis 3, wobei R² eine Alkyl-, Hydroxyalkyl- oder Alkoxyalkylgruppe ist, R⁵ eine Alkyl-, Hydroxyalkyl-, Alkoxyalkyl- oder Aralkylgruppe ist, An⁻ ist und Alkalimetall oder Ammoniumion, R⁶ und R⁷ jeweils unabhängig voneinander Alkyl- oder Alkenylgruppen und R³ eine C₁₀ bis C₃₀ Alkyl-, Alkenyl-, Alkaryl-, Aryl- oder Aralkylgruppe ist.

5. Zusammensetzung nach einem der Ansprüche 1 bis 4, wobei die Oxyalkylengruppe(n) OA Oxyethylen, Oxypropylen oder Gemische von Oxyethylen und Oxypropylengruppen ist(sind) und p von 1 bis 50 ist.

6. Zusammensetzung nach einem der Ansprüche 1 bis 5, wobei Link ist eine Gruppe nach einer der Formeln:
- CH₂-CHOH-CH₂-O-; -CH₂-CHOH-CH₂- (OA)ₚ-O-;
- CH₂-CHOH-CH₂-N (R⁶) -; oder -CH₂-CHOH-CH₂- (OA)ₚ-N (R⁷) -:
wobei OA, p, R⁶ und R⁷ wie entweder in Anspruch 1 oder Anspruch 2 definiert sind.

7. Zusammensetzung nach einem der Ansprüche 1 bis 6, wobei die agrochemisch aktive Verbindung ist ein oder mehrere Pflanzenwuchsregulatoren, Herbizide und/oder Pestizide, zum Beispiel Insektizide, Fungizide, Akarizide, Nematizide, Mitizide, Rodentizide, Bakterizide, Molluskizide und/oder Vogelabwehrmittel.

8. Zusammensetzung nach Anspruch 7, wobei die agrochemisch aktive Verbindung mindestens ein wasserlösliches Herbizid ist, oder enthält.

9. Zusammensetzung nach Anspruch 8, wobei das wasserlösliche Herbizid ist, oder enthält, mindestens ein Phosphonomethylglycin, besonders Glyphosat und/oder Sulfosat; mindestens eine Phosphinylaminosäure, besonders Glufosinat; und/oder mindestens eine Bipyridinium-Verbindung, besonders Paraquat.

10. Verbindung nach der allgemeinen Formel (IIa) :
R¹-(R²)X¹-[Link¹]-R³ wobei X¹, R¹, R² und R³ wie in einem der Ansprüche 1 bis 5 definiert sind und Link¹ ist eine verknüpfende Gruppe nach einer der Formeln:
- CH₂-CHOH-CH₂- (OA)ₚ-O-; -CH₂-CHOH-CH₂-N (R⁶) -;
oder -CH₂-CHOH-CH₂- (OA) ₚ-N (R⁷) -; wobei OA, p, R⁶ und R⁷ wie für Formel (I) in einem der Ansprüche 1 bis 5 definiert sind.

11. Verbindung nach Anspruch 10, wobei X¹ ist N; Link¹ ist nach Formel: -CH₂-CHOH-CH₂-(OA)ₚ-O-; und R¹ ist eine Polyhydroxyalkylgruppe mit einer linearen C₄- bis C₇-Kette und mindestens drei Hydroxygruppen, die direkt an die Kettenkohlenstoffatome gebunden sind.

12. Verbindung nach Anspruch 11, wobei die Gruppe R¹ ein Zuckerrest ist, besonders ein Monosaccharid wie Glucose, Fructose oder Sorbitol, ein Disaccharid wie Maltose oder Palitose oder ein höheres Oligosaccharid.

13. Verbindung nach der allgemeinen Formel (IIb) : R¹- (R²) X^{1a}- [Link²] -R³, wobei R¹, R² und R³ wie obenstehend für Formel (I) definiert sind; X^{1a} ist N⁺->O⁻, N⁺R⁴- oder R⁵An⁻ wobei: R⁴⁻, R⁵ und An⁻ wie oben für Formel (I) definiert sind; und Link² ist eine verknüpfende Gruppe nach einer der Formeln: -CH₂-CHOH-CH₂-O-;
- CH₂-CHOH-CH₂- (OA) ₚ-O-; -CH₂-CHOH-CH₂-N (R⁶) -; oder - CH₂-CHOH-CH₂-(OA)ₚ-N(R⁷)-; wobei OA, p, R⁶ und R⁷ wie für Formel (I) in einem der Ansprüche 1 bis 5 definiert sind.

14. Zusammensetzung nach Anspruch 1, wobei die Verbindung nach Formel (I) eine Verbindung nach Formel (IIa) wie in einem der Ansprüche 10 bis 12 definiert, oder eine Verbindung nach Formel (IIb) wie in Anspruch 13 definiert, ist.

15. Verfahren der Vegetationsbehandlung durch Anwenden einer Zusammensetzung nach einem der Ansprüche 1 bis 9 oder 14 an Pflanzen und/oder Boden.

16. Verfahren des Abtötens oder Inhibierens von Vegetation durch Anwenden einer Formulierung nach Anspruch 1, welche beinhaltet ein oder mehrere Wuchsregulatoren und/oder Herbizide und mindestens eine Verbindung nach der allgemeinen Formel (I) wie in einem der Ansprüche 1 bis 6 definiert oder nach Formel (IIa) wie in Anspruch 10 definiert oder Formel (IIb) wie in Anspruch 11 definiert, als Zusatzstoff.

17. Verfahren des Abtötens von Pflanzenschädlingen durch Anwenden einer Formulierung nach Anspruch 1, welche beinhaltet ein oder mehrere Pestizide, zum Beispiel Insektizide, Fungizide oder Akarizide, und mindestens eine Verbindung nach der allgemeinen Formel (I) wie in einem der Ansprüche 1 bis 6 definiert, oder nach der Formel (IIa) wie in Anspruch 10 definiert oder Formel (IIb) wie in Anspruch 11 definiert, als Zusatzstoff.

18. Verwendung einer Verbindung nach Formel (I) wie in einem der Ansprüche 1 bis 6 definiert, oder nach Formel (IIa) wie in Anspruch 10 definiert oder Formel (IIb) wie in Anspruch 11 definiert, als agrochemischen Zusatzstoff.

19. Verwendung nach Anspruch 18, wobei die agrochemisch aktive Verbindung ein oder mehrere Pflanzenwuchsregulatoren, Herbizide und/oder Pestizide, zum Beispiel Insektizide, Fungizide, Akarizide, Nematizide, Mitizide, Rodentizide, Bakterizide, Molluskizide und/oder Vogelabwehrmittel sind.

20. Verwendung nach Anspruch 19, wobei die agrochemisch aktive Verbindung mindestens ein wasserlösliches Herbizid ist, oder enthält.

21. Verwendung nach Anspruch 20, wobei die agrochemisch aktive Verbindung ist, oder enthält, mindestens ein Phosphonomethylglycin, besonders Glyphosat und/oder Sulfosat; mindestens eine Phosphinylaminosäure, besonders Glufosinat; und/oder mindestens eine Bipyridiniumverbindung, besonders Paraquat.

## Revendications

1. Composition agrochimique qui comprend un composé actif d'un point de vue agrochimique et un composé de formule (I) :
R¹-(R²)X¹-[Lien]-R³ (I)
dans laquelle
R¹ est un polyhydroxy hydrocarbyle ;
R² est H ou un groupe hydrocarbyle ou est un groupe tel que défini pour R¹ ;
X¹ est N ; N⁺→O⁻ ; N⁺R⁴⁻ dans laquelle : R⁴⁻ est un groupe hydrocarbyle en C₁ à C₆ portant un substituant anionique ; en particulier -CH₂-COO⁻ ; ou N⁺R⁵An⁻ dans laquelle : R⁵ est un groupe hydrocarbyle en C₁ à C₂₀ ; et An⁻ est un anion pour balance de charge ;
Lien est un groupe de liaison de formule : -CH₂-CHOH-X²-
dans laquelle X² est
une liaison directe ; -CH₂-O- ; -CH₂-N (R⁶) - ; -CH₂-(OA)ₚ-O ; ou -CH₂-(OA)ₚ-N(R⁷)- ;
dans laquelle
OA est un résidu oxyalkylène
p est de 1 à 100
R⁶ est H ; un groupe hydrocarbyle en C₁ à C₈ ; ou un groupe R¹- (R²) X¹-CH₂-CHOH-CH₂- dans laquelle R¹, R² et X¹ sont définis tels que ci-dessus ; et
R⁷ est H ; un groupe hydrocarbyle en C₁ à C₈ ; ou un groupe R¹-(R²) X¹-CH₂-CHOH-CH₂-(OA)ₚ- dans laquelle R¹, R², X¹, OA et p sont définis tels que ci-dessus ; et
R³ est un groupe hydrocarbyle.

2. Composition selon la revendication 1 dans laquelle R¹ est un groupe polyhydroxy alkyle ayant une chaîne linéaire en C₄ à C₇ et au moins trois groupes hydroxyles directement liés à des atomes de carbone de chaîne.

3. Composition selon la revendication 2 dans laquelle R¹ est un groupe de formule : -CH₂-(CHOH)₄-CH₂OH.

4. Composition selon l'une quelconque des revendications 1 à 3 dans laquelle R² est un groupe alkyle, hydroxyalkyle ou alkoxyalkyle, R⁵ est un groupe alkyle, hydroxyalkyle, alkoxyalkyle ou aralkyle, An- est un métal alcalin ou un ion ammonium, R⁶ et R⁷ sont chacun indépendamment des groupes alkyles ou alkényles et R³ est un groupe alkyle, alkényle, alkaryle, aryle ou aralkyle en C₁₀ à C₃₀.

5. Composition selon l'une quelconque des revendications 1 à 4 dans laquelle le(s) groupe(s) oxyalkylène (s) OA est(sont) des groupes oxyéthylènes, oxypropylènes ou des mélanges d'oxyéthylène et d'oxypropylène et p est de 1 à 50.

6. Composition selon l'une quelconque des revendications 1 à 5 dans laquelle Lien est un groupe d'une des formules : -CH₂-CHOH-CH₂-O- ; -CH₂-CHOH-CH₂-(OA)ₚ-O- ; -CH₂-CHOH-CH₂-N(R⁶)- ; ou -CH₂-CHOH-CH₂-(OA)ₚ-N(R⁷)- ; dans lesquelles OA, p, R⁶ et R⁷ sont tels que définis dans la revendication 1 ou la revendication 2.

7. Composition selon l'une quelconque des revendications 1 à 6 dans laquelle le composé actif d'un point de vue agrochimique est un ou plusieurs régulateur(s) de croissance, herbicide(s) et/ou pesticide(s), par exemple des insecticides, des fongicides, des acaricides, des nématocides, des miticides, des rodenticides, des bactéricides, des molluscicides et/ou des produits répulsifs pour les oiseaux.

8. Composition selon la revendication 7 dans laquelle le composé actif d'un point de vue agrochimique est ou comprend au moins un herbicide soluble dans l'eau.

9. Composition selon la revendication 8 dans laquelle l'herbicide soluble dans l'eau est ou comprend au moins une phosphonométhyl glycine, en particulier le Glyphosate et/ou le Sulfosate ; au moins un acide aminé phosphinyle, en particulier le Glufosinate ; et/ou au moins un composé bipyridinium, en particulier le Paraquat.

10. Composé de formule générale (IIa) : R¹- (R²) X¹-[Lien¹] -R³ dans laquelle X¹, R¹, R² et R³ sont définis tels que dans l'une quelconque des revendications 1 à 5 et Lien¹ est un groupe de liaison d'une des formules : - CH₂-CHOH-CH₂-(OA)ₚ-O- ; -CH₂-CHOH-CH₂-N (R⁶) - ; ou -CH₂-CHOH-CH₂-(OA)ₚ-N(R⁷)- ; dans lesquelles OA, p, R⁶ et R⁷ sont tels que définis pour la formule (I) à l'une quelconque des revendications 1 à 5.

11. Composé selon la revendication 10 dans lequel X¹ est N ; Lien¹ est de formule : -CH₂-CHOH-CH₂-(OA)ₚ-O-; et R¹ est un groupe polyhydroxy alkyle ayant une chaîne linéaire en C₄ à C₇ et au moins trois groupes hydroxyles directement liés à des atomes de carbone de chaîne.

12. Composé selon la revendication 11 dans lequel le groupe R¹ est un résidu d'un sucre, en particulier un monosaccharide tel que le glucose, le fructose ou le sorbitol, un disaccharide tel que le maltose ou le palitose ou un oligosaccharide à chaîne plus longue.

13. Composé de formule générale (IIb) : R¹- (R²) X^{1a}-[Lien²] -R³ dans laquelle R¹, R² et R³ sont tels que définis ci-dessus pour la formule (I) : X^{1a} est N+→O⁻, N⁺R⁴- ou R⁵An⁻ dans laquelle : R⁴⁻, R⁵ et An⁻ sont tels que définis ci-dessus pour la formule (I) ; et Lien² est un groupe de liaison d'une des formules :
- CH₂-CHOH-CH₂-O- ; -CH₂-CHOH-CH₂- (OA)ₚ-O-; -CH₂-CHOH-CH₂-N (R⁶) - ; ou -CH₂-CHOH-CH₂- (OA)ₚ-N (R⁷) -; dans lesquelles OA, p, R⁶ et R⁷ sont tels que définis pour la formule (I) à l'une quelconque des revendications 1 à 5.

14. Composition selon la revendication 1 dans laquelle le composé de formule (I) est un composé de formule (IIa) telle que définie à l'une quelconque des revendications 10 à 12 ou un composé de formule (IIb) telle que définie dans la revendication 13.

15. Procédé de traitement de la végétation en appliquant à des plantes et/ou au sol une composition selon l'une quelconque des revendications 1 à 9 ou 14.

16. Procédé d'élimination ou d'inhibition de la végétation en appliquant une formulation selon la revendication 1 qui comprend un ou plusieurs régulateur(s) de croissance et/ou herbicide(s) et au moins un composé de formule générale (I) telle que définie à l'une quelconque des revendications 1 à 6 ou de formule générale (IIa) telle que définie dans la revendication 10 ou de formule (IIb) telle que définie dans la revendication 11, en tant qu'adjuvant.

17. Procédé d'élimination d'organismes nuisibles aux plantes en appliquant une formulation selon la revendication 1 qui comprend un ou plusieurs pesticide(s), par exemple des insecticides, des fongicides ou des acaricides, et au moins un composé de formule générale (I) telle que définie à l'une quelconque des revendications 1 à 6, ou de formule (IIa) telle que définie dans la revendication 10 ou de formule (IIb) telle que définie dans la revendication 11, en tant qu'adjuvant.

18. Utilisation d'un composé de formule (I) telle que définie à l'une quelconque des revendications 1 à 6 ou de formule (IIa) telle que définie dans la revendication 10 ou de formule (IIb) telle que définie dans la revendication 11 en tant qu'adjuvant agrochimique.

19. Utilisation selon la revendication 18 dans laquelle le composé actif d'un point de vue agrochimique est un ou plusieurs régulateur(s) de croissance des plantes, herbicide(s) et/ou pesticide(s), par exemple des insecticides, des fongicides, des acaricides, des nématocides, des miticides, des rodenticides, des bactéricides, des molluscicides et/ou des produits répulsifs pour les oiseaux.

20. Utilisation selon la revendication 19 dans laquelle le composé actif d'un point de vue agrochimique est ou comprend au moins un herbicide soluble dans l'eau.

21. Utilisation selon la revendication 20 dans laquelle le composé actif d'un point de vue agrochimique est ou comprend au moins une phosphonométhyl glycine, en particulier le Glyphosate et/ou le Sulfosate ; au moins un acide aminé phosphinyle, en particulier le Glufosinate ; et/ou au moins un composé bipyridinium, en particulier le Paraquat.
